# EUROPEAN PATENT APPLICATION

(11) **EP 1 033 404 A1**
(43) Date of publication of application: **06.09.2000**
(21) Application number: 99101925.8
(22) Date of filing: 29.01.1999
(51) Int. Cl.: C12N 15/12, C07K 14/47, C07K 16/18, C12Q 1/68

(54) **New gene with down-regulated expression in metastatic human melanoma cells**

(71) Applicant: F. HOFFMANN-LA ROCHE AG, 4070 Basel (CH)
(72) Inventor: VAN MUIJEN, Goosen, N.P., 6525 GV Nijmegen (NL); ZENDMAN, Albert, J.W., 6641 EK Beuningen (NL)
(74) Representative: Schreiner, Siegfried, Dr.

(57) **Abstract**

An isolated nucleic acid molecule (TM7XN1) with the nucleic acid sequence SEQ ID NO:1 is down-regulated during tumor progression and/or metastasis.

SEQUENCE LISTING

(1) GENERAL INFORMATION:

(i) APPLICANT:
(A) NAME: F. Hoffmann-La Roche AG
(B) STREET: -
(C) CITY: Basle
(E) COUNTRY: Switzerland
(F) POSTAL CODE (ZIP): CH-4070
(G) TELEPHONE: (61) 688 11 11
(H) TELEFAX: (61) 688 13 95
(I) TELEX: 962292 hlr ch

(ii) TITLE OF INVENTION: New gene with down-regulated expression in
metastatic human melanoma cells and a protein coded
thereby, methods of production, and use therof

(iii) NUMBER OF SEQUENCES: 9

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30B (EPO)

(2) INFORMATION FOR SEQ ID NO: 1:

(i) SEQUENCE CHARACTERISTICS:
(A) LENGTH: 3845 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: 5'UTR
(B) LOCATION:1..316

(ix) FEATURE:
(A) NAME/KEY: sig_peptide
(B) LOCATION:317..391

(ix) FEATURE:
(A) NAME/KEY: CDS
(B) LOCATION:317..2380

(ix) FEATURE:
(A) NAME/KEY: mat_peptide
(B) LOCATION:392..2377
(D) OTHER INFORMATION:/product= "TM7XN1 protein"

(ix) FEATURE:
(A) NAME/KEY: protein_bind
(B) LOCATION:1949..1972
(D) OTHER INFORMATION:/bound_moiety= "tyrosine kinase"
/note= "putative"

(ix) FEATURE:
(A) NAME/KEY: misc_binding
(B) LOCATION:2339..2371
(D) OTHER INFORMATION:/bound_moiety= "AMP"
/note= "putative"

(ix) FEATURE:
(A) NAME/KEY: polyA_signal
(B) LOCATION:3793..3798

(ix) FEATURE:
(A) NAME/KEY: polyA_site
(B) LOCATION:3811

(xi) SEQUENCE DESCRIPTION: SEQ ID NO: 1:
GGGTACCGGG CCCCCCCTCG ACTCTAGCAT GCGCACAGCG GGCGCAGCAA CACGCTGGCA 60
GGTTAAGCCT CTGGGGGTGG ATCCTGAAAG GTGGTCCAGC CGCCTGGCCC TGCGTGGGAC 120
CCTCCACCTG GCAGCAGGTA CCCAAACAAG GGCTGGACAG CAGGGTCTCG CTCTGTCACA 180
CAGGCTGGAG TGCAGTGGTG TGATCTTGGC TCATCGTAAC CTCCACCTCC CGGGTTCAAG 240
TGATTCTCAT GCCTCAGCCT CCCGAGTAGC TGGGATTACA GGTGGTGACT TCCAAGAGTG 300
ACTCCGTCGG AGGAAA ATG ACT CCC CAG TCG CTG CTG CAG ACG ACA CTG 349
TTC CTG CTG AGT CTG CTC TTC CTG GTC CAA GGT GCC CAC GGC AGG GGC 397
CAC AGG GAA GAC TTT CGC TTC TGC AGC CAG CGG AAC CAG ACA CAC AGG 445
AGC AGC CTC CAC TAC AAA CCC ACA CCA GAC CTG CGC ATC TCC ATC GAG 493
AAC TCC GAA GAG GCC CTC ACA GTC CAT GCC CCT TTC CCT GCA GCC CAC 541
CCT GCT TCC CGA TCC TTC CCT GAC CCC AGG GGC CTC TAC CAC TTC TGC 589
CTC TAC TGG AAC CGA CAT GCT GGG AGA TTA CAT CTT CTC TAT GGC AAG 637
CGT GAC TTC TTG CTG AGT GAC AAA GCC TCT AGC CTC CTC TGC TTC CAG 685
CAC CAG GAG GAG AGC CTG GCT CAG GGC CCC CCG CTG TTA GCC ACT TCT 733
GTC ACC TCC TGG TGG AGC CCT CAG AAC ATC AGC CTG CCC AGT GCC GCC 781
AGC TTC ACC TTC TCC TTC CAC AGT CCT CCC CAC ACG GCC GCT CAC AAT 829
GCC TCG GTG GAC ATG TGC GAG CTC AAA AGG GAC CTC CAG CTG CTC AGC 877
CAG TTC CTG AAG CAT CCC CAG AAG GCC TCA AGG AGG CCC TCG GCT GCC 925
CCC GCC AGC CAG CAG TTG CAG AGC CTG GAG TCG AAA CTG ACC TCT GTG 973
AGA TTC ATG GGG GAC ATG GTG TCC TTC GAG GAG GAC CGG ATC AAC GCC 1021
ACG GTG TGG AAG CTC CAG CCC ACA GCC GGC CTC CAG GAC CTG CAC ATC 1069
CAC TCC CGG CAG GAG GAG GAG CAG AGC GAG ATC ATG GAG TAC TCG GTG 1117
CTG CTG CCT CGA ACA CTC TTC CAG AGG ACG AAA GGC CGG AGG GGG GAG 1165
GCT GAG AAG AGA CTC CTC CTG GTG GAC TTC AGC AGC CAA GCC CTG TTC 1213
CAG GAC AAG AAT TCC AGC CAC GTC CTG GGT GAG AAG GTC TTG GGG ATT 1261
GTG GTA CAG AAC ACC AAA GTA GCC AAC CTC ACG GAG CCC GTG GTG CTC     1309
ACC TTC CAG CAC CAG CTA CAG CCG AAG AAT GTG ACT CTG CAA TGT GTG     1357
TTC TGG GTT GAA GAC CCC ACA TTG AGC AGC CCG GGG CAT TGG AGC AGT     1405
GCT GGG TGT GAG ACC GTC AGG AGA GAA ACC CAA ACA TCC TGC TTC TGC     1453
AAC CAC TTG ACC TAC TTT GCA GTG CTG ATG GTC TCC TCG GTG GAG GTG     1501
GAC GCC GTG CAC AAG CAC TAC CTG AGC CTC CTC TCC TAC GTG GGC TGT     1549
GTC GTC TCT GCC CTG GCC TGC CTT GTC ACC ATT GCC GCC TAC CTC TGC     1597
TCC AGG AGG AAA CCT CGG GAC TAC ACC ATC AAG GTG CAC ATG AAC CTG     1645
CTG CTG GCC GTC TTC CTG CTG GAC ACG AGC TTC CTG CTC AGC GAG CCG     1693
GTG GCC CTG ACA GGC TCT GAG GCT GGC TGC CGA GCC AGT GCC ATC TTC     1741
CTG CAC TTC TCC CTG CTC ACC TGC CTT TCC TGG ATG GGC CTC GAG GGG     1789
TAC AAC CTC TAC CGA CTC GTG GTG GAG GTC TTT GGC ACC TAT GTC CCT     1837
GGC TAC CTA CTC AAG CTG AGC GCC ATG GGC TGG GGC TTC CCC ATC TTT     1885
CTG GTG ACG CTG GTG GCC CTG GTG GAT GTG GAC AAC TAT GGC CCC ATC     1933
ATC TTG GCT GTG CAT AGG ACT CCA GAG GGC GTC ATC TAC CCT TCC ATG 1981
TGC TGG ATC CGG GAC TCC CTG GTC AGC TAC ATC ACC AAC CTG GGC CTC 2029
TTC AGC CTG GTG TTT CTG TTC AAC ATG GCC ATG CTA GCC ACC ATG GTG 2077
GTG CAG ATC CTG CGG CTG CGC CCC CAC ACC CAA AAG TGG TCA CAT GTG 2125
CTG ACA CTG CTG GGC CTC AGC CTG GTC CTT GGC CTG CCC TGG GCC TTG 2173
ATC TTC TTC TCC TTT GCT TCT GGC ACC TTC CAG CTT GTC GTC CTC TAC 2221
CTT TTC AGC ATC ATC ACC TCC TTC CAA GGC TTC CTC ATC TTC ATC TGG 2269
TAC TGG TCC ATG CGG CTG CAG GCC CGG GGT GGC CCC TCC CCT CTG AAG 2317
AGC AAC TCA GAC AGC GCC AGG CTC CCC ATC AGC TCG GGC AGC ACC TCG 2365
TCC AGC CGC ATC TAG GCCTCCAGCC CACCTGCCCA TGTGATGAAG CAGAGATGCG 2420
GCCTCGTCGC ACACTGCCTG TGGCCCCCGA GCCAGGCCCA GCCCCAGGCC AGTCAGCCGC 2480
AGACTTTGGA AAGCCCAACG ACCATGGAGA GATGGGCCGT TGCCATGGTG GACGGACTCC 2540
CGGGCTGGGC TTTTGAATTG GCCTTGGGGA CTACTCGGCT CTCACTCAGC TCCCACGGGA 2600
CTCAGAAGTG CGCCGCCATG CTGCCTAGGG TACTGTCCCC ACATCTGTCC CAACCCAGCT 2660
GGAGGCCTGG TCTCTCCTTA CAACCCCTGG GCCCAGCCCT CATTGCTGGG GGCCAGGCCT 2720
TGGATCTTGA GGGTCTGGCA CATCCTTAAT CCTGTGCCCC TGCCTGGGAC AGAAATGTGG 2780
CTCCAGTTGC TCTGTCTCTC GTGGTCACCC TGAGGGCACT CTGCATCCTC TGTCATTTTA 2840
ACCTCAGGTG GCACCCAGGG CGAATGGGGC CCAGGGCAGA CCTTCAGGGC CAGAGCCCTG 2900
GCGGAGGAGA GGCCCTTTGC CAGGAGCACA GCAGCAGCTC GCCTACCTCT GAGCCCAGGC 2960
CCCCTCCCTC CCTCAGCCCC CCAGTCCTCC CTCCATCTTC CCTGGGGTTC TCCTCCTCTC 3020
CCAGGGCCTC CTTGCTCCTT CGTTCACAGC TGGGGGTCCC CGATTCCAAT GCTGTTTTTT 3080
GGGGAGTGGT TTCCAGGAGC TGCCTGGTGT CTGCTGTAAA TGTTTGTCTA CTGCACAAGC 3140
CTCGGCCTGC CCCTGAGCCA GGCTCGGTAC CGATGCGTGG GCTGGGCTAG GTCCCTCTGT 3200
CCATCTGGGC CTTTGTATGA GCTGCATTGC CCTTGCTCAC CCTGACCAAG CACACGCCTC 3260
AGAGGGGCCC TCAGCCTCTC CTGAAGCCCT CTTGTGGCAA GAACTGTGGA CCATGCCAGT 3320
CCCGTCTGGT TTCCATCCCA CCACTCCAAG GACTGAGACT GACCTCCTCT GGTGACACTG 3380
GCCTAGAGCC TGACACTCTC CTAAGAGGTT CTCTCCAAGC CCCCAAATAG CTCCAGGCGC 3440
CCTCGGCCGC CCATCATGGT TAATTCTGTC CAACAAACAC ACACGGGTAG ATTGCTGGCC 3500
TGTTGTAGGT GGTAGGGACA CAGATGACCG ACCTGGTCAC TCCTCCTGCC AACATTCAGT 3560
CTGGTATGTG AGGCGTGCGT GAAGCAAGAA CTCCTGGAGC TACAGGGACA GGGAGCCATC 3620
ATTCCTGCCT GGGAATCCTG GAAGACTTCC TGCAGGAGTC AGCGTTCAAT CTTGACCTTG 3680
AAGATGGGAA GGATGTTCTT TTTACGTACC AATTCTTTTG TCTTTTGATA TTAAAAAGAA 3740
GTACATGTTC ATTGTAGAGA ATTTGGAAAC TGTAGAAGAG AATCAAGAAG AAAAATAAAA 3800
ATCAGCTGTT GTAAAAAAAA AAAAAAAAAA AAAAAAAAAA AAAAA 3845

(2) INFORMATION FOR SEQ ID NO: 2:

(i) SEQUENCE CHARACTERISTICS:
(A) LENGTH: 688 amino acids
(B) TYPE: amino acid
(D) TOPOLOGY: linear

(ii) MOLECULE TYPE: protein

(xi) SEQUENCE DESCRIPTION: SEQ ID NO: 2:

(2) INFORMATION FOR SEQ ID NO: 3:

     (i) SEQUENCE CHARACTERISTICS:
          (A) LENGTH: 21 base pairs
          (B) TYPE: nucleic acid
          (C) STRANDEDNESS: single
          (D) TOPOLOGY: linear

    (ii) MOLECULE TYPE: other nucleic acid
          (A) DESCRIPTION:  /desc = "beta2-microglobulin :
  sense primer"

    (xi) SEQUENCE DESCRIPTION: SEQ ID NO: 3:

CTCGCGCTAC TCTCTCTTTC T                                                21

(2) INFORMATION FOR SEQ ID NO: 4:

     (i) SEQUENCE CHARACTERISTICS:
          (A) LENGTH: 21 base pairs
          (B) TYPE: nucleic acid
          (C) STRANDEDNESS: single
          (D) TOPOLOGY: linear

    (ii) MOLECULE TYPE: other nucleic acid
          (A) DESCRIPTION:  /desc = "beta2-microglobulin :
  antisense primer"

    (xi) SEQUENCE DESCRIPTION: SEQ ID NO: 4:

TGTCGGATTG ATGAAACCCA G                                                21

(2) INFORMATION FOR SEQ ID NO: 5:

     (i) SEQUENCE CHARACTERISTICS:
          (A) LENGTH: 18 base pairs
          (B) TYPE: nucleic acid
          (C) STRANDEDNESS: single
          (D) TOPOLOGY: linear

    (ii) MOLECULE TYPE: other nucleic acid
          (A) DESCRIPTION:  /desc = "TM7XN1 cDNA : sense
          primer"

(xi) SEQUENCE DESCRIPTION: SEQ ID NO: 5:

CCATCTTTCT GGTGACGC 18

(2) INFORMATION FOR SEQ ID NO: 6:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "TM7XN1 cDNA : antisense
primer"

(xi) SEQUENCE DESCRIPTION: SEQ ID NO: 6:

GAGCTGATGG GGAGCCTG 18

(2) INFORMATION FOR SEQ ID NO: 7:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "TM7XN1 DNA : sense primer"

(xi) SEQUENCE DESCRIPTION: SEQ ID NO: 7:

GCTCTGTCTC TCGTGGTC 18

(2) INFORMATION FOR SEQ ID NO: 8:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "TM7XN1 DNA : antisense
primer"

(xi) SEQUENCE DESCRIPTION: SEQ ID NO: 8:

GCATGGTCCA CAGTTCTTG 19

(2) INFORMATION FOR SEQ ID NO: 9:

(i) SEQUENCE CHARACTERISTICS:
(A) LENGTH: 10 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "AP7 : primer"

(xi) SEQUENCE DESCRIPTION: SEQ ID NO: 9:

TCGATACAGG 10

SEQUENCE LISTING

<210> 1
<211> 3845
<212> DNA
<213> Homo sapiens

<220>
<221> 5'UTR
<222> (1)..(316)

<220>
<221> sig_peptide
<222> (317)..(391)

<220>
<221> CDS
<222> (317)..(2380)

<220>
<221> mat_peptide
<222> (392)..(2377)
<223> TM7XN1 protein

<220>
<221> protein_bind
<222> (1949)..(1972)

<220>
<221> misc_binding
<222> (2339)..(2371)

<220>
<221> polyA_signal
<222> (3793)..(3798)

<220>
<221> polyA_site
<222> (3811)

<400> 1
gggtaccggg ccccccctcg actctagcat gcgcacagcg ggcgcagcaa cacgctggca 60
ggttaagcct ctgggggtgg atcctgaaag gtggtccagc cgcctggccc tgcgtgggac 120
cctccacctg gcagcaggta cccaaacaag ggctggacag cagggtctcg ctctgtcaca 180
caggctggag tgcagtggtg tgatcttggc tcatcgtaac ctccacctcc cgggttcaag 240
tgattctcat gcctcagcct cccgagtagc tgggattaca ggtggtgact tccaagagtg 300
actccgtcgg aggaaa atg act ccc cag tcg ctg ctg cag acg aca ctg ttc 352
ctg ctg agt ctg ctc ttc ctg gtc caa ggt gcc cac ggc agg ggc cac 400
agg gaa gac ttt cgc ttc tgc agc cag cgg aac cag aca cac agg agc 448
agc ctc cac tac aaa ccc aca cca gac ctg cgc atc tcc atc gag aac 496
tcc gaa gag gcc ctc aca gtc cat gcc cct ttc cct gca gcc cac cct 544
gct tcc cga tcc ttc cct gac ccc agg ggc ctc tac cac ttc tgc ctc 592
tac tgg aac cga cat gct ggg aga tta cat ctt ctc tat ggc aag cgt 640
gac ttc ttg ctg agt gac aaa gcc tct agc ctc ctc tgc ttc cag cac 688
cag gag gag agc ctg gct cag ggc ccc ccg ctg tta gcc act tct gtc 736
acc tcc tgg tgg agc cct cag aac atc agc ctg ccc agt gcc gcc agc 784
ttc acc ttc tcc ttc cac agt cct ccc cac acg gcc gct cac aat gcc 832
tcg gtg gac atg tgc gag ctc aaa agg gac ctc cag ctg ctc agc cag 880
ttc ctg aag cat ccc cag aag gcc tca agg agg ccc tcg gct gcc ccc 928
gcc agc cag cag ttg cag agc ctg gag tcg aaa ctg acc tct gtg aga 976
ttc atg ggg gac atg gtg tcc ttc gag gag gac cgg atc aac gcc acg 1024
gtg tgg aag ctc cag ccc aca gcc ggc ctc cag gac ctg cac atc cac 1072
tcc cgg cag gag gag gag cag agc gag atc atg gag tac tcg gtg ctg 1120
ctg cct cga aca ctc ttc cag agg acg aaa ggc cgg agg ggg gag gct 1168
gag aag aga ctc ctc ctg gtg gac ttc agc agc caa gcc ctg ttc cag 1216
gac aag aat tcc agc cac gtc ctg ggt gag aag gtc ttg ggg att gtg 1264
gta cag aac acc aaa gta gcc aac ctc acg gag ccc gtg gtg ctc acc 1312
ttc cag cac cag cta cag ccg aag aat gtg act ctg caa tgt gtg ttc 1360
tgg gtt gaa gac ccc aca ttg agc agc ccg ggg cat tgg agc agt gct 1408
ggg tgt gag acc gtc agg aga gaa acc caa aca tcc tgc ttc tgc aac 1456
cac ttg acc tac ttt gca gtg ctg atg gtc tcc tcg gtg gag gtg gac 1504
gcc gtg cac aag cac tac ctg agc ctc ctc tcc tac gtg ggc tgt gtc 1552
gtc tct gcc ctg gcc tgc ctt gtc acc att gcc gcc tac ctc tgc tcc 1600
agg agg aaa cct cgg gac tac acc atc aag gtg cac atg aac ctg ctg 1648
ctg gcc gtc ttc ctg ctg gac acg agc ttc ctg ctc agc gag ccg gtg 1696
gcc ctg aca ggc tct gag gct ggc tgc cga gcc agt gcc atc ttc ctg 1744
cac ttc tcc ctg ctc acc tgc ctt tcc tgg atg ggc ctc gag ggg tac 1792
aac ctc tac cga ctc gtg gtg gag gtc ttt ggc acc tat gtc cct ggc 1840
tac cta ctc aag ctg agc gcc atg ggc tgg ggc ttc ccc atc ttt ctg 1888
gtg acg ctg gtg gcc ctg gtg gat gtg gac aac tat ggc ccc atc atc 1936
ttg gct gtg cat agg act cca gag ggc gtc atc tac cct tcc atg tgc 1984
tgg atc cgg gac tcc ctg gtc agc tac atc acc aac ctg ggc ctc ttc 2032
agc ctg gtg ttt ctg ttc aac atg gcc atg cta gcc acc atg gtg gtg 2080
cag atc ctg cgg ctg cgc ccc cac acc caa aag tgg tca cat gtg ctg 2128
aca ctg ctg ggc ctc agc ctg gtc ctt ggc ctg ccc tgg gcc ttg atc 2176
ttc ttc tcc ttt gct tct ggc acc ttc cag ctt gtc gtc ctc tac ctt 2224
ttc agc atc atc acc tcc ttc caa ggc ttc ctc atc ttc atc tgg tac 2272
tgg tcc atg cgg ctg cag gcc cgg ggt ggc ccc tcc cct ctg aag agc 2320
aac tca gac agc gcc agg ctc ccc atc agc tcg ggc agc acc tcg tcc 2368
agc cgc atc tag gcctccagcc cacctgccca tgtgatgaag cagagatgcg 2420
gcctcgtcgc acactgcctg tggcccccga gccaggccca gccccaggcc agtcagccgc 2480
agactttgga aagcccaacg accatggaga gatgggccgt tgccatggtg gacggactcc 2540
cgggctgggc ttttgaattg gccttgggga ctactcggct ctcactcagc tcccacggga 2600
ctcagaagtg cgccgccatg ctgcctaggg tactgtcccc acatctgtcc caacccagct 2660
ggaggcctgg tctctcctta caacccctgg gcccagccct cattgctggg ggccaggcct 2720
tggatcttga gggtctggca catccttaat cctgtgcccc tgcctgggac agaaatgtgg 2780
ctccagttgc tctgtctctc gtggtcaccc tgagggcact ctgcatcctc tgtcatttta 2840
acctcaggtg gcacccaggg cgaatggggc ccagggcaga ccttcagggc cagagccctg 2900
gcggaggaga ggccctttgc caggagcaca gcagcagctc gcctacctct gagcccaggc 2960
cccctccctc cctcagcccc ccagtcctcc ctccatcttc cctggggttc tcctcctctc 3020
ccagggcctc cttgctcctt cgttcacagc tgggggtccc cgattccaat gctgtttttt 3080
ggggagtggt ttccaggagc tgcctggtgt ctgctgtaaa tgtttgtcta ctgcacaagc 3140
ctcggcctgc ccctgagcca ggctcggtac cgatgcgtgg gctgggctag gtccctctgt 3200
ccatctgggc ctttgtatga gctgcattgc ccttgctcac cctgaccaag cacacgcctc 3260
agaggggccc tcagcctctc ctgaagccct cttgtggcaa gaactgtgga ccatgccagt 3320
cccgtctggt ttccatccca ccactccaag gactgagact gacctcctct ggtgacactg 3380
gcctagagcc tgacactctc ctaagaggtt ctctccaagc ccccaaatag ctccaggcgc 3440
cctcggccgc ccatcatggt taattctgtc caacaaacac acacgggtag attgctggcc 3500
tgttgtaggt ggtagggaca cagatgaccg acctggtcac tcctcctgcc aacattcagt 3560
ctggtatgtg aggcgtgcgt gaagcaagaa ctcctggagc tacaggacag gggagccatc 3620
attcctgcct gggaatcctg gaagacttcc tgcaggagtc agcgttcaat cttgaccttg 3680
aagatgggaa ggatgttctt tttacgtacc aattcttttg tcttttgata ttaaaaagaa 3740
gtacatgttc attgtagaga atttggaaac tgtagaagag aatcaagaag aaaaataaaa 3800
atcagctgtt gtaaaaaaaa aaaaaaaaaa aaaaaaaaaa aaaaa 3845

<210> 2
<211> 687
<212> PRT
<213> Homo sapiens

<400> 2


<210> 3
<211> 21
<212> DNA
<213> Artificial Sequence

<220>
<223> Description of Artificial
      Sequence:beta2-microglobulin : sense primer

<400> 3
ctcgcgctac tctctctttc t                                              21


<210> 4
<211> 21
<212> DNA
<213> Artificial Sequence

<220>
<223> Description of Artificial
      Sequence:beta2-microglobulin : antisense primer

<400> 4
tgtcggattg atgaaaccca g                                                21


<210> 5
<211> 18
<212> DNA
<213> Artificial Sequence

<220>
<223> Description of Artificial Sequence:TM7XN1 cDNA :
      sense primer

<400> 5
ccatctttct ggtgacgc                                                    18


<210> 6
<211> 18
<212> DNA
<213> Artificial Sequence

<220>
<223> Description of Artificial Sequence:TM7XN1 cDNA :
      antisense primer

<400> 6
gagctgatgg ggagcctg                                                    18


<210> 7
<211> 18
<212> DNA
<213> Artificial Sequence

<220>
<223> Description of Artificial Sequence:TM7XN1 DNA :
      sense primer

<400> 7
gctctgtctc tcgtggtc                                                    18


<210> 8
<211> 19
<212> DNA
<213> Artificial Sequence

<220>
<223> Description of Artificial Sequence:TM7XN1 DNA :
      antisense primer

<400> 8
gcatggtcca cagttcttg                                                   19

<210> 9
<211> 10
<212> DNA
<213> Artificial Sequence

<220>
<223> Description of Artificial Sequence:AP7 : primer

<400> 9
tcgatacagg                                                                10

## Description

The present invention relates to the new gene TM7XN1 which is down-regulated in metastasis, a protein coded thereby, their use for diagnostics and therapeutics, especially in the field of cancer. In particular, the invention relates to the diagnosis of said gene TM7XN1 in mammalian, especially in melanoma cells, and to gene therapy methods to inhibit TM7XN1 in its function in tumor cells.

Melanoma is one of the most aggressive human malignant tumors known. Although this type of skin cancer is not very frequent, a worrisome aspect is its rise in incidence. So far radical excision is the therapy of choice, but only successful if the tumor has not yet disseminated. Therefore, early diagnosis and assessment of the progression stage is very important. To be able to improve such a classification, molecular markers are crucial.

Up to now a great number of genes or gene-products have been identified which are differentially expressed during melanocytic tumor progression (Weterman, M.A., et al., Lab. Invest. 70 (1994) 593-608). To identify these changes several strategies have been used both on DNA, mRNA and protein level. The last decade extensive attention has been paid to the detection of differentially expressed mRNAs. Among the strategies used to detect either up- or downregulated sequences, differential hybridization, subtractive hybridization, mRNA Differential Display and Serial Analysis of Gene Expression (Velculescu, V.E., et al., Science 270 (1995) 484-487) are the most common. In oncobiology these techniques have been successfully used in the identification and subsequent isolation of differentially expressed genes in several types of tumors (including melanoma) (Shen, R., et al., Proc. Natl. Acad. Sci. USA 92 (1995) 6778-6782; Lee, J.H., et al., Int. J. Cancer 71 (1997) 1035-1044; Zhang, L., et al., Sciene 276 (1997) 1268-1272; van Groningen, J.J., et al., Cancer Res. 55 (1995) 6237-6243; Yeatman, T.J., et al., Nucleic Acids 23 (1995) 4007-4008; Jiang, H., et al., Oncogene 11 (1995) 2477-2486; Francia, G., et al., Cancer Res. 56 (1996) 3855-3858; Duncan, L.M., et al., Cancer Res. 58 (1998) 1515-1520).

To identify and isolate gene-products that may be involved in melanocytic tumor progression mRNA Differential Display which was first described by Liang and Pardee (Liang, P., and Pardee, A.B., Science 257 (1992) 967-971) was performed.

Metastasis of cancer is a phenomenon which requires several hurdles to be taken such as degradation of the extracellular matrix and basal membrane, intra- and extravasation of vessels of the blood and of the lymphatic system, escape by the attack of the immune system and homing and colonization of distant organs (Pardee, A.B., Advances in Cancer Res. 65 (1994) 213-227; Ponta, H., et al., Biochem. Biophys. Acta 1198 (1994) 1-10). A further level of complexity is achieved by the finding that different types of cancers make use of different molecular mechanisms for metastasis and exhibit different tropism of metastasis.

Cell adhesion molecules play an important role in the invasion, dissemination, extravasation and lodging of tumor cells. The interaction of disseminated tumor cells with endothelium and tissue stroma is supposed to be one of the critical steps in tumor progression and metastasis formation (Ebnet, K., et al., Annu. Rev. Immunol. 14 (1996) 155-177; Varner, J.A., and Cheresh, D.A., Curr. Opin. Cell Biol. 8 (1996) 724-730; Albelda, S.M., Lab. Invest. 68 (1993) 4-17).

It is an object of the invention to provide new genes involved in metastasis. The invention describes the cloning of a protein, termed TM7XN1, which is down-regulated in metastatic cancer cells, especially in melanoma cells, as compared to their non-metastatic counterparts. TM7XN1 may be involved in promotion of several steps of the metastatic cascade due to its down-regulation.

The invention therefore comprises an isolated nucleic acid molecule (TM7XN1) with down-regulated expression in metastatic tumor cells, preferably in melanoma cells, and which is involved in inducing tumor progression and/or metastasis, especially in malignant melanoma cells, wherein the nucleic acid is selected from the group consisting of
a) a nucleic acid having the sequence SEQ ID NO:1,
b) a nucleic acid which hybridizes under stringent conditions with a nucleic acid fragment coding for amino acids 2-315 of said nucleic acid of a) or the complementary fragment or
c) a nucleic acid which due to the degeneracy of the genetic code encodes a polypeptide encoded by one of the foregoing nucleic acids.

The invention further comprises a recombinant polypeptide which is coded by the nucleic acid sequences according to the invention, preferably by the DNA sequence shown in SEQ ID NO:1, as well as such nucleic acid molecules per se .

The polypeptide can be defined by its DNA sequence and by the amino acid sequence derived therefrom which preferably has the amino acid sequence SEQ ID NO:2 or a fragment thereof, preferably with aa 1-315. The isolated TM7XN1 polypeptide can occur in natural allelic variations which differ from individual to individual. Such variations of the amino acids are usually amino acid substitutions. However, they may also be deletions, insertions or additions of amino acids to the total sequence. The TM7XN1 protein according to the invention - depending, both in respect of the extent and type, on the cell and cell type in which it is expressed- can be in glycosylated or non-glycosylated form. Polypeptides with anti-metastatic activity and nucleic acids coding therefor can be identified by transfection of TM7XN1-negative metastasizing melanoma cells with expression vectors for TM7XN1, establishment of stable transfectants and evaluation of in vitro invasiveness in Matrigel invasion assays and their metastatic capacity after xenografting into nude mice.

"Polypeptide with TM7XN1 activity or TM7XN1" means also proteins with minor amino acid variations but with substantially the same TM7XN1 activity. Substantially the same means that the activities are of the same biological properties and the polypeptides show preferably at least 75% homology (identity) to the amino acid sequence. More preferably, the amino acid sequences are at least 90% identical.

The term "nucleic acid molecule or nucleic acid" denotes a polynucleotide molecule which can be, for example, a DNA, RNA, PNA or derivatized active DNA or RNA. DNA and/or RNA molecules are preferred, however.

The term "hybridize under stringent conditions" means that two nucleic acid fragments are capable of hybridization to one another under standard hybridization conditions described in Sambrook et al., Molecular Cloning: A laboratory manual (1989) Cold Spring Harbor Laboratory Press, New York, USA.

More specifically, the term "stringent conditions" as used herein refers to hybridization in 6.0 x SSC at about 45°C, followed by a wash with 2.0 x SSC at 50°C. For selection of the stringency the salt concentration in the wash step can be selected, for example from about 2.0 x SSC at 50°C, for low stringency, to about 0.2 x SSC at 50°C, for high stringency. In addition, the temperature in the wash step can be increased from low stringency conditions at room temperatures, about 22°C, to high stringency conditions at about 65°C.

The term "isolated" as used throughout this application refers to a nucleic acid or polypeptide having a TM7XN1 activity and is substantially free of cellular material or culture medium, when produced by recombinant DNA techniques, or chemical precursors or other chemicals, when synthesized chemically. An isolated nucleic acid is preferably free of sequences which naturally flank the nucleic acid (i.e. sequences located at the 5' and the 3' ends of the nucleic acid) in the organism from which the nucleic acid is derived.

TM7XN1 can be purified after recombinant production by affinity chromatography using known protein purification techniques, including immunoprecipitation, gel filtration, ion exchange chromatography, chromatofocussing, isoelectric focussing, selective precipitation, electrophoresis, or the like.

The polypeptides according to the invention can be produced by recombinant means, or synthetically. Non-glycosylated TM7XN1 polypeptide is obtained when it is produced recombinantly in prokaryotes. With the aid of the nucleic acid sequences provided by the invention it is possible to search for the TM7XN1 gene or its variants in genomes of any desired cells (e.g. apart from human cells, also in cells of other mammals), to identify these and to isolate the desired gene coding for the TM7XN1 protein. Such processes and suitable hybridization conditions are known to a person skilled in the art and are described, for example, by Sambrook et al., Molecular Cloning: A laboratory manual (1989) Cold Spring Harbor Laboratory Press, New York, USA, and Hames, B.D., Higgins, S.G., Nucleic acid hybridisation - a practical approach (1985) IRL Press, Oxford, England. In this case the standard protocols described in these publications are usually used for the experiments.

The use of recombinant DNA technology enables the production of numerous active TM7XN1 derivatives. Such derivatives can, for example, be modified in individual or several amino acids by substitution, deletion or addition. The derivatization can, for example, be carried out by means of site directed mutagenesis. Such variations can be easily carried out by a person skilled in the art (J. Sambrook, B.D. Hames, loc. cit.). It merely has to be ensured that the characteristic properties of TM7XN1 are preserved. The invention therefore in addition concerns a TM7XN1 polypeptide which is a product of prokaryotic or eukaryotic expression of an exogenous nucleic acid molecule according to the invention.

With the aid of such nucleic acids coding for a TM7XN1 protein, the protein according to the invention can be obtained in a reproducible manner and in large amounts. For expression in prokaryotic or eukaryotic organisms, such as prokaryotic host cells or eukaryotic host cells, the nucleic acid is integrated into suitable expression vectors, according to methods familiar to a person skilled in the art. Such an expression vector preferably contains a regulatable/inducible promoter. These recombinant vectors are then introduced for the expression into suitable host cells such as, e.g., E. coli as a prokaryotic host cell or Saccharomyces cerevisiae, Teratocarcinoma cell line PA-1 sc 9117 (Büttner et al., Mol. Cell. Biol. 11 (1991) 3573-3583), insect cells, CHO or COS cells as eukaryotic host cells and the transformed or transduced host cells are cultured under conditions which allow expression of the heterologous gene. The isolation of the protein can be carried out according to known methods from the host cell or from the culture supernatant of the host cell. Such methods are described for example by Ausubel I., Frederick M., Current Protocols in Mol. Biol. (1992), John Wiley and Sons, New York. Also in vitro reactivation of the protein may be necessary if it is not found in soluble form in the cell culture.

The invention further comprises recombinant expression vectors which are suitable for the expression of TM7XN1, recombinant host cells transfected with such expression vectors, as well as a process for the recombinant production of a protein which is encoded by the TM7XN1 gene.

The invention further comprises a method for detecting a nucleic acid molecule of gene TM7XN1, comprising incubating a sample (e.g., body fluids such as blood, cell lysates) with the isolated nucleic acid molecule according to the invention and determining hybridization under stringent conditions of said isolated nucleic acid molecule to a target nucleic acid molecule for determination of presence of a nucleic acid molecule which is the TM7XN1 gene and therefore a method for the identification of the metastatic potential and/or progression of tumor cells, preferably of melanoma cells.

On the basis of the nucleic acids provided by the invention it is possible to provide a test which can be used to detect nucleic acids with down-regulated expression in metastatic human tumor cells. Such a test can be carried out by means of nucleic acid diagnostics. In this case the sample to be examined, preferably a melanoma cells or a nucleic acid containing an extract from a melanoma cell, is contacted with a probe that is selected from the group comprising
a) the nucleic acids coding for the polypeptide of SEQ ID NO:2 or a nucleic acid sequence which is complementary thereto, or
b) nucleic acids which hybridize under stringent conditions with a nucleic acid fragment coding for amino acids 2-315 of said nucleic acid or (a) or the complementary fragment, wherein
the nucleic acid probe is incubated with the nucleic acid of the sample and the hybridization is detected optionally by means of a further binding partner for the nucleic acid of the sample and/or the nucleic acid probe.

Preferably, as a probe a nucleic acid fragment coding for a part or all of the polypeptide fragment 2-315 is used.

Hybridization between the probe and nucleic acids from the sample indicates the presence of the RNA of such proteins. Such methods are known to a person skilled in the art and are described, for example, in WO 89/06698, EP-A 0 200 362, USP 2915082, EP-A 0 063 879, EP-A 0 173 251, EP-A 0 128 018.

In a preferred embodiment of the invention the coding nucleic acid of the sample is amplified before the test, for example by means of the known PCR technique. Usually a derivatized (labeled) nucleic acid probe is used within the framework of nucleic acid diagnostics. This probe is contacted with a denatured DNA or RNA from the sample which is bound to a carrier and in this process the temperature, ionic strength, pH and other buffer conditions are selected - depending on the length and composition of the nucleic acid probe and the resulting melting temperature of the expected hybrid - such that the labeled DNA or RNA can bind to homologous DNA or RNA (hybridization see also Wahl, G.M., et al., Proc. Natl. Acad. Sci. USA 76 (1979) 3683-3687). Suitable carriers are membranes or carrier materials based on nitrocellulose (e.g., Schleicher and Schüll, BA 85, Amersham Hybond, C.), strengthened or bound nitrocellulose in powder form or nylon membranes derivatized with various functional groups (e.g., nitro groups) (e.g., Schleicher and Schüll, Nytran; NEN, Gene Screen; Amersham Hybond M.; Pall Biodyne).

Hybridizing DNA or RNA is then detected by incubating the carrier with an antibody or antibody fragment after thorough washing and saturation to prevent unspecific binding. The antibody or the antibody fragment is directed towards the substance incorporated during derivatization into the nucleic acid probe. The antibody is in turn labeled. However, it is also possible to use a directly labeled DNA. After incubation with the antibodies it is washed again in order to only detect specifically bound antibody conjugates. The determination is then carried out according to known methods by means of the label on the antibody or the antibody fragment.

The detection of the expression can be carried out for example as:
- in situ hybridization with fixed whole cells, with fixed tissue smears and isolated metaphase chromosomes,
- colony hybridization (cells) and plaque hybridization (phages and viruses),
- Southern hybridization (DNA detection),
- Northern hybridization (RNA detection),
- serum analysis (e.g., cell type analysis of cells in the serum by slot-blot analysis),
- after amplification (e.g., PCR technique).

Therefore the invention also includes a method for the detection of the metastatic potential of melanoma cells, comprising
a) incubating a sample of body fluid of a patient suffering from cancer, especially of melanoma, melanoma cancer cells of a patient or of a cell extract or cell culture supernatants of said melanoma cancer cells, whereby said sample contains nucleic acids, with a nucleic acid probe which is selected from the group consisting of
   (i) the nucleic acid shown in SEQ ID NO:1 or a nucleic acid which is complementary thereto and
   (ii) nucleic acids which hybridize with a nucleic acid fragment coding for amino acids 2-315 of said nucleic acid of (a) or the complementary nucleic acid under stringent conditions, and
b) detecting hybridization by means of a further binding partner of the nucleic acid of the sample and/or the nucleic acid probe or by X-ray radiography.

Preferably the nucleic acid probe is incubated with the nucleic acid of the sample and the hybridization is detected optionally by means of a further binding partner for the nucleic acid of the sample and/or the nucleic acid probe.

The nucleic acids according to the invention are hence valuable prognostic markers in the diagnosis of the metastatic and progression potential of tumor cells, preferably melanoma cells, of a patient.

The invention further comprises a method for producing a protein whose expression is correlated with tumor metastasis, by expressing an exogenous DNA in prokaryotic or eukaryotic host cells and isolation of the desired protein, wherein the protein is coded by the nucleic acid molecules according to the invention, preferably by the DNA sequence shown in SEQ ID NO:1.

The protein can be isolated from the cells or the culture supernatant and purified by chromatographic means, preferably by ion exchange chromatography, affinity chromatography and/or reverse phase HPLC.

The invention further comprises an isolated protein according to the invention which is encoded by a nucleic acid molecule according to the invention, preferably having the nucleotide sequence set forth in SEQ ID NO:1.

The present invention relates to the cloning and characterization of the gene TM7XN1, which is down-regulated during tumor progression. The function of the gene according to the invention (TM7XN1) is to promote contact inhibition and anchorage dependence in tumor cells and its down-regulation promotes other essential steps of the metastatic cascade. Therefore the down-regulation of TM7XN1 gene correlates with a more aggressive behavior of the tumor cells and also with the potential of the formation of metastasis.

According to the invention up-regulators for the expression of TM7XN1 can be used to inhibit tumor progression/metastasis, preferably of malignant melanomas and mammary carcinomas, in vivo, preferably by somatic gene therapy.

The gene according to the invention is inversely correlated with metastatic potential of eight different human melanoma cell lines after subcutaneous inoculation into nude mice.

The 3845 basepair cDNA of TM7XN1 contains an ORF which codes for a protein of 687 amino acids (signal peptide: aa -25- -1, mature protein: aa 1-662 of SEQ ID NO:2). A variable poly-A tail can be the explanation for the difference in length between the estimated 4.2 kb on Northern and the 3845 bp full length cDNA. TM7XN1 is a novel integral seven transmembrane protein with a long N-terminal extracellular domain which, based on its homology, can be placed in the fast growing family of G-protein-coupled peptide hormone receptors, also known as the secretin family. Within this group recently an EGF-TM7 subfamily was described (McKnight, A.J., and Gordon, S., Immunol. Today 17 (1996) 283-287), of which several splice variants with variable numbers of EGF-binding domains have been reported (McKnight, A.J., and Gordon, S., J. Leukoc. Biol. 63 (1998) 271-280). In addition several other secretin receptor-like TM7 molecules with long N-terminal domains have been described, but all lacked EGF-binding domains (Tensen, C.P., et al., Proc. Natl. Acad. Sci. USA 91 (1994) 4816-4820; Osterhoff, C., et al., DNA Cell Biol. 16 (1997) 379-389; Nishimori, H., et al., Oncogene 15 (1997) 2145-2150; Shiratsuchi, T., et al., Cytogenet. Cell Genet. 79 (1997) 103-108; Lelianova, V.G., et al., J. Biol. Chem. 272 (1997) 21504-21508). Instead of EGF-binding domains some of these molecules possess other protein-binding domains in their long N-terminus. These dual-function proteins are believed to be formed by genetic rearrangements, like exon-shuffling, by which TM7 proteins gained protein-binding domains from other genes (Probst, W.C., et al., DNA Cell Biology 11 (1992) 1-20; van Lier, R.A., et al., Immunol. Lett. 54 (1996) 185-187; Hamann, J., et al., Genomics 32 (1996) 144-147).

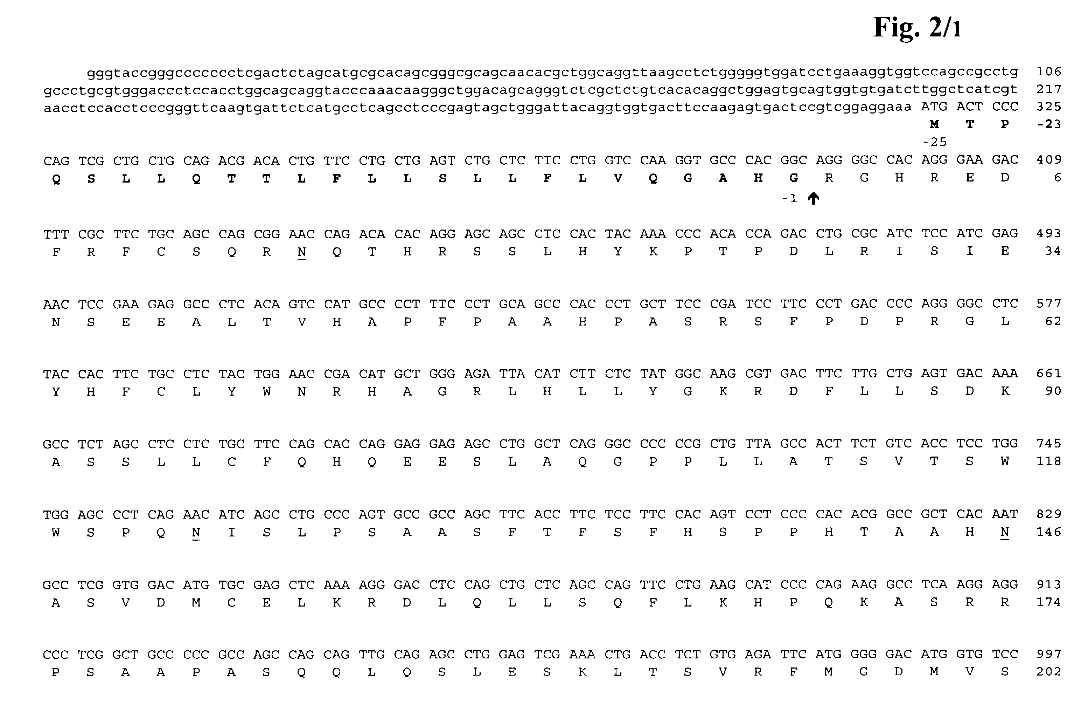

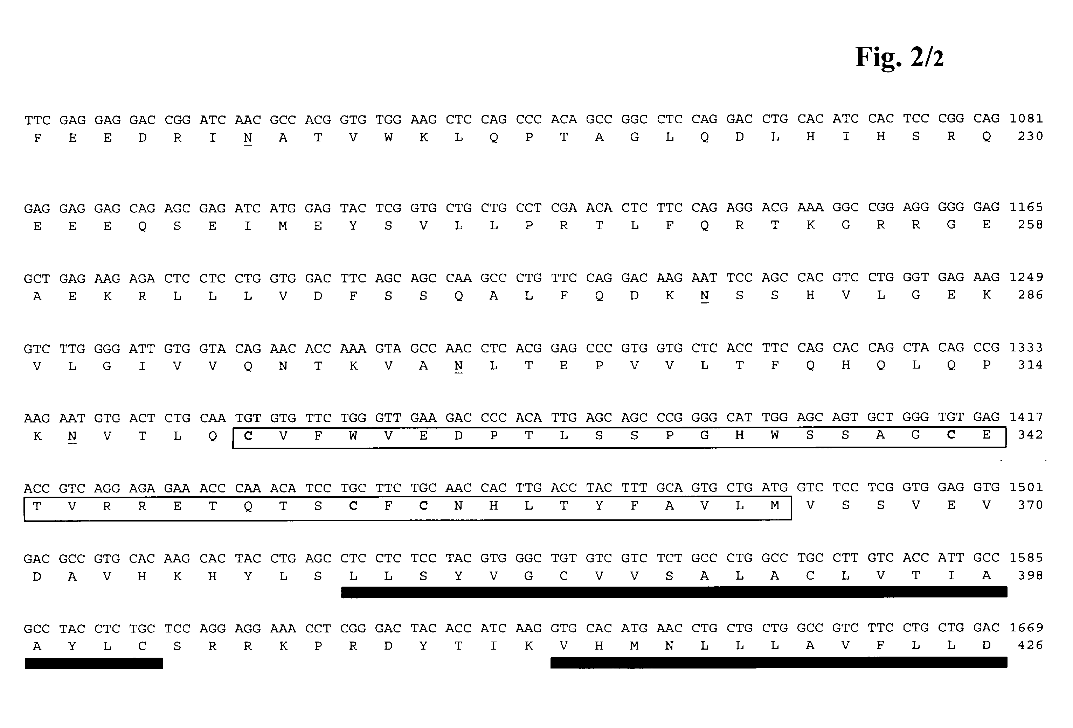

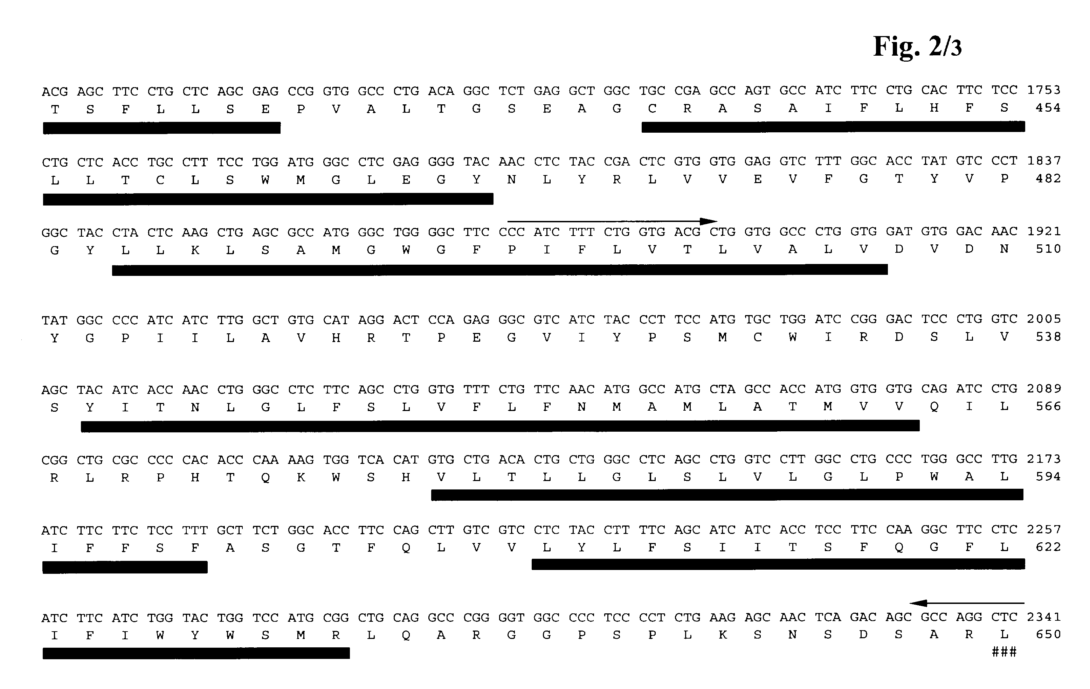

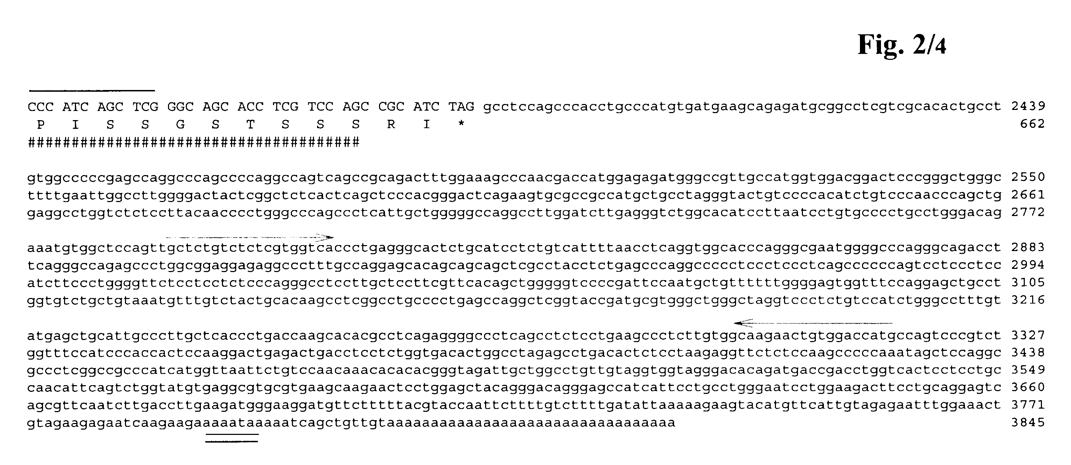

The TM7XN1 protein consists of several domains (Fig. 2). A signal sequence (aa -25- -1) is followed by a domain which may have a tertiary structure mainly determinated by the several cysteins present. This region is followed by a mucin-like, highly glycosylated spacer without cysteins. Just before the first TM a so-called cystein-box is situated (aa 321-364). The N-terminal domain including the cys-box spans aa 2-379. C-terminally seven transmembrane domains (aa 380-402; 414-433; 444-467; 485-506; 540-563; 578-599 and 609-631) and a cytoplasmic tail (aa 632-662) are situated. This cytoplasmic tail harbors an AMP-binding site that together with the phosphorylation sites may be indicative for interaction with components involved in intracellular signaling pathways. To gain insight into the relation of TM7XN1 with other homologues genes, TM7XN1 was compared and aligned with two members of the secretin receptor family, with all described members of the EGF-TM7 family, with all previously described non-EGF-binding domain containing secretin receptor homologues with long N-termini, and with several other homologues found during homology searches. Based on these comparisons, a novel LN-TM7 subfamily of the secretin family of G-protein-coupled receptors can be delineated. These molecules are identified by a C-terminal TM7 domain, homologues to the secretin receptor combined with a N-terminal extracellular domain of a length of at least 300 bp. In contrast, N-termini of secretin-like peptide hormone receptors are smaller than 200 bp. It was already postulated that the long N-termini capture a ligand and then present this ligand to the binding pocket situated inside the TM7 structure. The peptide hormone receptors do not need such a long N-terminus because of the small peptide ligand (Tensen, C.P., et al., Proc. Natl. Acad. Sci. USA 91 (1994) 4816-4820). Additional information that can be used as indication for future assignment and positioning of these proteins can come from the highly conserved cys-box (C-x₂-W-x₆₋₁₄-G-x-W-x₃-GC-x₉-T-x-C-x-C-x-HL-x₂-FAVLM), which is absent in the original peptide hormone receptors of the secretin family, and the LN-TM7 specific conserved residues described above. This cys-box is also found in non-TM7 proteins like the soluble rho-binding citron (Madaule, P., et al., FEBS Lett. 377 (1995) 243-248), and F31D5.4. F31D5.4, like the TM7XN1 homologue F31D5.5, also is a protein from C.elegans cosmid F31D5. The lower homology of BOSS and GRL101 may indicate a longer evolutionary distance.

However, homology to an extent of about 30% is only found in the TM7 part of the molecules. The N-terminal part without the cys-box, aa 1-315 or 25-315 (without signal peptide), and/or the C-terminal tail (638-652) are unique and characteristic of the proteins according to the invention and do not show homologies with other human proteins. Therefore, a preferred TM7XN1 polypeptide fragment starts with aa 1-25 and ends with an amino acid at approximately as 315. If transmembrane anchorage is desired, the polypeptide contains additionally an appropriate transmembrane and, preferably, a cytoplasmic domain.

Expression of the best studied EGF-TM7s is mainly restricted to cells of the haematopoietic lineage and solid-tumor derived cell lines (McKnight, A.J., and Gordon, S., Immunol. Today 17 (1996) 283-287). They are described as leukocyte antigens with a possible immunologic role. Recently CD97 expression has also been described in thyroid tumors (Aust, G. et al., Cancer Res. 57 (1997) 1798-1806). LN-TM7s have a somewhat broader expression pattern. As for CD97, expression in (para)thyroid tumors is also the case for KIAA0279 (AA843289; EMBL), which like TM7XN1 is mainly expressed in testis, brain, kidney, and prostate (Hamann, J., et al., J. Immunol. 155 (1995) 1942-1950). Human EST homologues of latrophilin are found in testis (AA778576; EMBL), and again parathyroid tumors (AA843854/AA705981; EMBL). In rat, latrophilin is also expressed in brain (Lelianova, V.G., et al., J. Biol. Chem. 272 (1997) 21504-21508). The BAI-genes are expressed in brain and to a lesser extent in heart. The GRL101 expression pattern is also BAI-like, being active in neurons of the central nervous system and also in the heart. So far, HE6 is only detected in the epididymal duct of the testis (Osterhoff, C., et al., DNA Cell Biol. 16 (1997) 379-389).

Based on the localization on 16q13, TM7XN1 is a candidate-gene for the tumor suppressor gene cyld1, which is involved in cylindromatosis. Cylindromatosis is a rare autosomal dominant skin disease, which forms benign neoplasms of the skin appendages. This cyld1 gene was localized to chromosome 16q12-q13 (Biggs, P.J., et al., Oncogene 12 (1996) 1375-1377).

Concluding, TM7XN1 is a novel protein placed in a novel LN-TM7 family of seven transmembrane proteins with long N-terminal domain. LN-TM7 molecules are G-protein-coupled membrane-anchored proteins with a long external tail. The functional domains, which are mostly protein binding, are presented to the environment by a long rod-like glycosylated spacer. The whole extracellular domain seems to be carried and stabilized by a cystein-box, serving as a statue-foot. In this way adhesional activity can be coupled to intracellular signaling.

The invention further provides methods for identifying and isolation of agonists of TM7XN1 or up-regulators for the expression of TM7XN1. Such agonists and up-regulators can be used to inhibit tumor progression or metastasis and cause massive apoptosis of tumor cells in vivo, preferably by somatic gene therapy.

According to the invention there are provided methods for identifying and isolation of compounds which have utility in the treatment of cancer, especially in inhibiting metastasis and related disorders. These methods include methods for modulating the expression of the polypeptides according to the invention, methods for identifying compounds which can selectively bind to the proteins according to the invention, and methods of identifying compounds which can modulate the activity of said polypeptides. The methods further include methods for modulating, preferably inducing, the transcription of TM7XN1 gene to mRNA, which preferably down-regulates the metastatic potential of a tumor cell. These methods can be conducted in vitro and vivo and may make use of and establish cell lines and transgenic animal models of the invention.

A TM7XN1 agonist is defined as a substance or compound which increases the biological activity of TM7XN1, a polypeptide and/or activates the transcription or translation of TM7XN1 gene. In general, screening procedures for TM7XN1 agonists involve contacting candidate substances with host cells in which invasiveness is mediated by down-regulated expression of TM7XN1 under conditions favorable for measuring TM7XN1 activity and/or immunological assays (ELISAs) with anti-TM7XN1-antibodies and/or TM7XN1 protein or protein fragments.

TM7XN 1 activity may be measured in several ways. Typically, the activation is apparent by a change in cell physiology, such as decreased mobility and invasiveness in vitro, or by a change in the differentiation rate, or by a change in cell metabolism leading to decreased proliferation.

The TM7XN1 gene and protein of the invention can be used to identify and design drugs which interfere with proliferation and dissemination of tumor cells.

The purification of the TM7XN1 protein is preferably done by affinity chromatography using anti-TM7XN1-antibodies. Such antibodies can be obtained in a simple manner according to known methods by using the protein according to the invention as an antigen or an immunogen. The invention therefore in addition concerns the use of TM7XN1 protein for the production of antibodies. Anti-TM7XN1 antibodies are produced by immunization and appropriate vertebrate host with purified TM7XN1 or polypeptide derivatives of TM7XN1, preferably with an adjuvant. Such peptide fragments are preferably fragments from the characteristic protein regions of TM7XN1, which are preferably aa 1-315 and aa 638-652 having a length of 5-300 aa. Said techniques are well-known in the literature and are described, for example, by Harlow and Lane eds., Antibodies: A laboratory manual (1988), Cold Spring Harbor Laboratories Press.

For this, animals which are usually used for this purpose, such as, in particular, sheep, rabbits, or mice, are immunized with the protein according to the invention or a preferred fragment, and subsequently the antiserum is isolated from the immunized animals according to known methods or spleen cells of the immunized animals are fused with immortalized cells, such as, e.g., myeloma cells, according tothe method of Kohler and Milstein, Nature 256 (1975) 495-497. Those cells which produce a monoclonal antibody against the TM7XN1 protein are selected from the hybridoma cells obtained in this way and cloned. The monoclonal or polyclonal antibodies obtained in this way can be bound to a support material, such as, e.g., cellulose, for an immunoabsorptive purification of TM7XN1. Furthermore, antibodies of this kind can be used for the detection of TM7XN1 in samples, such as, e.g., cut tissue or body fluids, preferably for the determination of malignant diseases, most preferably for the metastatic potential of tumor cells. In such assays, TM7XN1 is bound immunologically to its antibody in the specific step. The invention therefore concerns specific antibodies against the TM7XN1 protein which are obtainable by immunizing an animal with said TM7XN1 protein and isolating the antibodies from the serum or spleen cells of the immunized animals, and their use for the determination of TM7XN1.

The antibodies according to the invention are preferably used for immunohistochemistry on negative and positive melanoma cells and Western blots with such cells.

The following examples, references, sequence listing and figures are provided to aid the understanding of the present invention, the true scope of which is set forth in the appended claims. It is understood that modifications can be made in the procedures set forth without departing from the spirit of the invention.

### Description of the Figures and the Sequences

- **Figure 1**: **A**: Differential display gel of cell lines 1F6 and 1F6m using primer pair T₁₂MG and AP₇ (5'-TCGATACAGG-3' - SEQ ID NO:9). Clone 25 (arrow), a 250 bp cDNA, is much more abundant in IF6. (Four cutting marks surrounding this band are still visible.)
**B**: Northern blot analysis of clone 25 on a panel of eight human melanoma cell lines with different metastatic capacity after subcutaneous inoculation into nude mice (Westphal, J.R., et al., Br. J. Cancer 76 (1997) 561-570).
- **Figure 2**: Full-length cDNA (SEQ ID NO:1) and deduced protein sequence (SEQ ID NO:2) of TM7XN1 (clone 25). The protein contains a signal sequence (fat) plus cleavable site (fat arrow), several N-linked glycosylation sites (N), a cystein box (open box), seven transmembrane domains (gray boxes), an AMP-binding site (###), and a stopcodon (asterisk). Polyadenylation signal (double underlined), cDNA primers (closed arrow), and intron-spanning primers (open arrow) are also depicted.
- **Figure 3**: **A**: Expression of TM7XN1 in human melanoma cell lines with different metastatic potential using RT-PCR on total RNA of our panel of eight human melanoma cell lines. First lane shows the DNA molecular weight marker XIV (100 bp ladder, Boehringer Mannheim GmbH, DE).
**B**: β₂-Microglobulin control RT-PCR.
- **Figure 4**: Schematic overview of the domains in the TM7XN1 protein.
- **SEQ ID NO:1**: cDNA and amino acid sequence of TM7XN1.
- **SEQ ID NO:2**: Amino acid of TM7XN1
- **SEQ ID NO:3-9**: Primer sequences

### Materials and Methods

### Melanoma Cell Lines

Twenty-three human melanoma cell lines were used. A panel of eight melanoma cell lines containing 530, 1F6, M14, Me157, BLM, MV3, MV1, and 1F6m, a metastatic subline of 1F6, was described earlier (Westphal, J.R., et al., Br. J. Cancer 76 (1997) 561-570). In this panel of cell lines 530 and 1F6 are poorly metastatic cell lines, while MV3 and BLM represent the group of highly metastatic cell lines. MV1, M14 and Me157 are cell lines with an intermediate metastatic capacity. 1F6m can be placed between the intermediate and highly metastatic cell lines. Other human melanoma cell lines used were: 451Lu, 518A2, 603, A375m, A375p, Bowes, BRO, E10, M24met, MKR, OCM1, OMMI, SKmel28, WM164 and ZKR. As non-melanoma cell lines we used cell lines from diverse tumor origin: A431, CaCo2, Daudi, HepG2, HT29, HT1080, Jurkat, K562, Molt4, U937, Umscc2 and U20S. The origin of most cell lines was previously described by de Vries et al. (de Vries, T.J., et al., Cancer Res. 57 (1997) 3223-3229). Other previously described cell lines are A431, HepG2, HT29, HT1080, K562 and U937 (Quax, P.H., et al., Cancer Res. 50 (1990) 1488-1494), BRO (Lockshin, A., et al., Cancer Res. 45 (1985) 345-450), OCM1 (Danen, E.H., et al., Melanoma Res. 6 (1996) 31-35), and OMM1 (Luyten, G.P., et al., Int. J. Cancer 66 (1996) 380-387). Cell lines 451Lu and WM164 (Juhasz, I., et al., Am. J. Pathol. 143 (1993) 528-537), as well as MKR and ZKR (Scheibenbogen, C., et al., Int. J. Cancer 54 (1993) 494-498) were kindly provided from the institutions where the cell lines were established. Cell lines A375m, A375p, A431, Bowes, CaCo2, Daudi, Jurkat, Molt4, and Skmel28 were obtained from the American Type Culture Collection (Rockville, MD, US).

All cell lines were grown in Dulbecco's modified Eagle's medium (BioWhittaker, Walkersville, MD, US) or RPMI (BioWhittaker) supplemented with 10% FCS (Fetal Calf Serum) (BioWhittaker) and antibiotics. The cultures were maintained at 5% CO₂ and 37°C in a humidified atmosphere.

Primary cell cultures, including melanocytes, nevus cells, fibroblasts, keratinocytes, endothelial cells, pericytes, smooth muscle cells, and dendritic cells were cultured in vitro under their specific conditions. Fibroblasts were grown on Eagle's Minimum Essential Medium (BioWhittaker) supplemented with 20% human serum, 10 % FCS, and antibiotics. Keratinocytes were cultured on keratinocyte serum free medium (Gibco, Grant Island, NY, US) supplemented with 50 mg bovine pituitary extract per liter (Gibco), 5 µg human EGF (Epidermal Growth Factor) per liter (Gibco), 2 mM glutamin, 1 mM pyruvate, and antibiotics. Melanocytes, and nevus cells were grown on HAM's F10 medium (BioWhittaker) supplemented with 2% Ultroser-G synthetic serum (Gibco), 0.1 mM IBMX (3' -isobutyl-1-methyl-xanthine)(Sigma, St. Louis, MO), 10 ng per liter PMA (phorbol-12-myristate-14-acetate)(Sigma), 2 mM glutamin, 1 mM pyruvate, and antibiotics.

### Human Melanocytic Lesions

Lesions from all stages of melanocytic tumor progression were excised from patients at the University Hospital Nijmegen, The Netherlands. From each excised lesion a paracentral slice was taken and after removal of most of the surrounding skin and fat, they were snapfrozen in liquid nitrogen and stored at -80°C until use.

### RNA Isolation

From cultured cells total RNA was isolated using the RNeasy kit (Qiagen, Hilden, DE) following the manufacturers protocol. From tissue samples total RNA was isolated by disrupting about 25 frozen sections of 20 µm thickness in 1 ml RNAzolB™ (Campro, Veenendaal, NL) using a pestle. After completion of the RNAzolB™ method, RNA samples were purified from melanin (pigment) and DNA using the RNeasy kit.

### mRNA Differential Display

Before starting Differential Display PCR, DNaseI treatment was performed on the RNA samples using the Message-Clean™ kit (GenHunter Corporation, Brookline, US). For Differential Display the RNAmap™ protocol was used with some minor modifications. In short, RT-PCR (Reverse Transcriptase-PCR) was performed on 200 ng of DNase treated total RNA (1xRT buffer: 125 mM Tris-HCl pH 8.3, 188 mM KCl, 7.5 mM MgCl₂, 25 mM dithiothreitol) in the presence of 1 µM T₁₂MN primer, 20 µM dNTP mix, and 100 Units of MoMLV reverse transcriptase (Life Technologies, Rockville, MD, US). One tenth of the cDNA was amplified in the following PCR (1xPCR buffer: 10 mM Tris-HCl pH 9.0, 1.5 mM MgCl₂) in the presence of 1 µM T₁₂MN primer, 2 µM dNTP-dATP mix, 500 nM arbitrary primer (AP), 1 Unit of AmpliTaq™ DNA polymerase (Perkin-Elmer, Branchburg, NJ, US), and 5 µCi of [α-³²P]-dATP (3000 mCi/mmol; Amersham, Little Chalfont, UK). Differing from the original protocol, [³²P]-dATP was used instead of [³⁵S]-dATP. For the PCR, combinations of the four T₁₂MN primers together with six arbitrary primers, AP_{1,2,6,7,11,and 12} (Bauer, D., et al., Nucleic Acids Res. 21 (1993) 4272-4280), were used. All PCR reactions were carried out in a Thermocycler 480 (Perkin-Elmer, Branchburg, NJ, US). Forty cycles of PCR (30" at 94°C, 2' at 40°C and 30" at 72°C) were preceded by 5' denaturation at 94°C and followed by 5' elongation at 72°C. Of the PCR products 3.5 µl were mixed with 2 µl of loading buffer and separated on a 6% denaturing sequencing gel. Gels were dried and autoradiographed using Kodak Xomat-S films.

### cDNA Isolation and Amplification

Differentially expressed cDNA bands were cut out of the gel and cDNA was eluted from the gel and reamplified. Conditions of reamplification are the same as those of the Differential Display PCR except that no radioactive dATP was added, and a normal dNTP mixture was used. Reamplified cDNAs were checked on a 2% agarose gel containing ethidium bromide. PCR products of the expected size were purified from the gel with a DNA Clean-up kit (Promega, Madison, WI) and used as probes for Northern blot analyses.

### cDNA Cloning and selection

Differential cDNAs were cloned into a pCRII TA-cloning vector (Invitrogen, Madison, WI) and false positives were identified using a dot-blot selection method (Callard, D., et al., Biotechniques 16 (1994) 1096-1097; Callard, D., et al., Biotechniques 16 (1994) 1100-1103). In this way the most positive, differentially expressed cDNAs were selected. Control restriction analysis (EcoRI flanking the insert sites) was also carried out to check for length of the insert.

### Northern blot analysis

Ten micrograms of total RNA were treated with glyoxal/DMSO (Sambrook et al., Molecular Cloning: A laboratory manual (1989) Cold Spring Harbor Laboratory Press, New York, USA), separated on a 1.2% agarose gel and blotted onto a Hybond N⁺ membrane (Amersham, Aylesbury, UK). cDNA probes were radiolabeled by [³²P]-dATP incorporation using a random-primed DNA labeling kit (Boehringer Mannheim GmbH, Mannheim, DE). Membranes were hybridized overnight with the radiolabeled probes at 65°C in a hybridization mix (0.25 M sodium phosphate buffer pH 7.2, 7% SDS, 1% BSA, 1 mM EDTA, 0.1 mg/ml single stranded Salmon sperm DNA). Afterwards membranes were washed at 65°C with wash buffers containing decreasing amounts of salt (1% SDS, 1mM EDTA and 0.25-0.05 M sodium phosphate pH 7.2), and autoradiographed using Kodak Xomat-S films.

### cDNA Library Screening

cDNA probes were labeled as described before and hybridized (Sambrook et al., Molecular Cloning: A laboratory manual (1989) Cold Spring Harbor Laboratory Press, New York, USA) to a λZAP cDNA library of human melanoma cell line 530.

### RT-PCR

To study mRNA expression of the isolated clone in various human cell types, tumor cell lines, and in human melanocytic lesions RT-PCR was performed. The cDNA synthesis (10' at 25°C, followed by 59' at 42°C) was performed on 0.5-1.0 pg of total RNA using the AMV RT kit (Boehringer Mannheim GmbH, Penzberg, DE). The reaction was supplemented with 0.04 Units of random hexadeoxynucleotide primers, 2 µl 25mM MgCl₂, 1 µl 10 mM dNTPs, 1 µl of RT buffer (100 mM Tris/HCl pH8.3, 500 µM KCl), 25 Units RNasin, 10 Units AMV reverse transcriptase and water to a final volume of 10 µl. For amplification one tenth of the cDNA was supplemented with 2.5 µl of PCR-buffer (200 mM (NH₄)₂SO₄, 750 mM Tris/HCl pH 9,0.1% Tween), 5 µl 1M dNTPs, 10 pmoles of each primer, 2.5 µl 15 mM MgCl₂, 0.15 Units of Thermoperfectplus™ DNA polymerase (Integro, Zaandam, NL) and water to a final volume of 25 µl . The PCR conditions were 45" at 94°C, 1' at 59°C and 1'30" at 72°C for 30 cycles. These cycles were preceded by 3' denaturation at 94°C and followed by a 5' elongation step at 72°C. All amplifications were performed in an Eppendorf Mastercycler 5330 (Eppendorf, Hamburg, DE). Primer combinations and PCR product lengths were:

ß2-Microglobulin:	sense:	5'-CTCGCGCTACTCTCTCTTTCT-3'	(SEQ ID NO:3)
antisense:	5'-TGTCGGATTGATGAAACCCAG-3'	(SEQ ID NO:4)
cDNA product length: 136 bp

TM7XN1 cDNA:	sense:	5'-CCATCTTTCTGGTGACGC-3'	(SEQ ID NO:5)
antisense:	5'-GAGCTGATGGGGAGCCTG-3'	(SEQ ID NO:6)
cDNA product length: 474 bp

### Chromosomal localization

Chromosomal localization of the corresponding gene was determined by genomic PCR on a panel of hamster/human and mouse/human hybrid cell lines (Geurts van Kessel, A.H., et al., Proc. Natl. Acad. Sci. USA 80 (1983) 3748-3752). DNA of these cell lines, each specific for one human chromosome, was kindly provided by Dr. A. Simons, Department of Human Genetics, University Hospital Nijmegen, The Netherlands. PCR conditions and program were the same as used for the cDNA primer combinations described above. Intronspanning primers used:

TM7XN1 DNA:	sense:	5'-GCTCTGTCTCTCGTGGTC-3'	(SEQ ID NO:7)
antisense:	5'-GCATGGTCCACAGTTCTTG-3'	(SEQ ID NO:8)
DNA product length: 528 bp

### DNA Sequencing and Homology Search

After transfection and growth in E.coli JM109 cells, cloned re-amplified PCR fragments were purified with the Wizard miniprep kit (Promega, Madison, WI) and sequenced using the Amplicycle™ sequence kit (Perkin-Elmer, Branchburg, US). Homology searches were performed using BLAST (Altschul, S.F., et al., J. Mol. Biol. (1990) 403-410) and other software on all kinds of public servers of DNA and protein databases on Internet: (http://www.genome.ad.jp/; http://www.ncbi.nlm.nih.gov/; http://expasy.hcuge.ch/; http://dot.imgen.bcm.tmc.edu:9331/; http://www.genome.wi.mit.edu/).

### Example 1

### Isolation of differentially expressed cDNAs

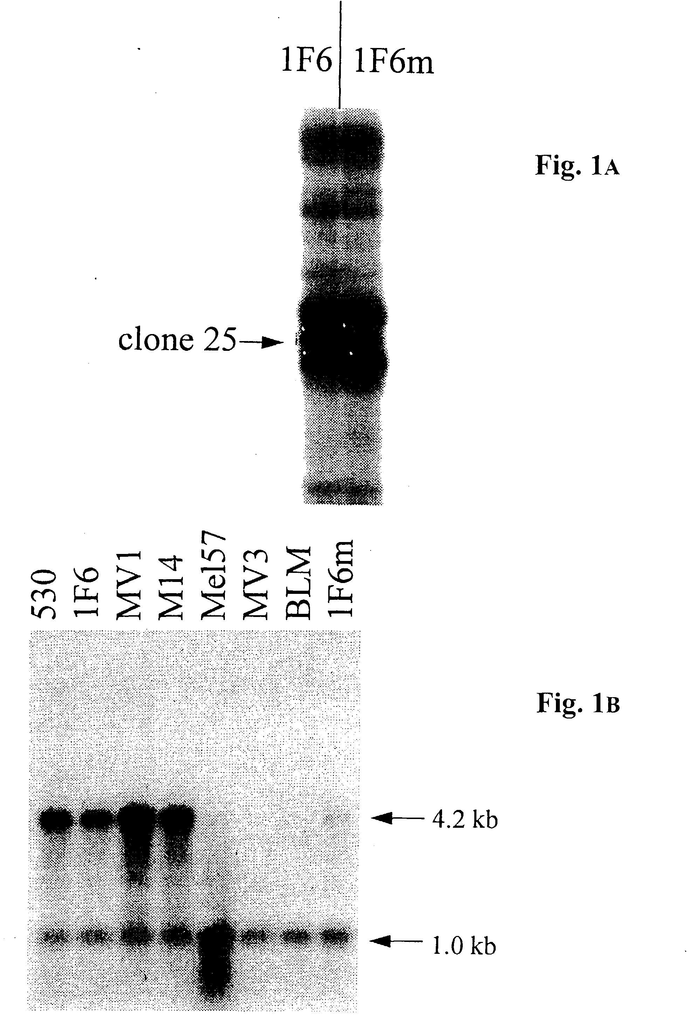

In the search for differential mRNA expression between human melanoma cell lines 1F6 and its metastatic variant 1F6m, 24 primer combinations (4 T₁₂MN and 6 APs) were used in the Differential Display. There were detected about 30 differential cDNAs (Fig. 1A). All cDNAs were routinely cloned into a TA-cloning vector. After hybridization on a Northern blot containing RNA from the panel of human melanoma cell lines with varying metastatic potential, three differentially expressed clones remained. One of the cDNAs, clone 25, revealed two bands at 4.2 kb and 1.0 kb (Fig. 1B). Marked expression of the 4.2 kb transcript was only present in the poorly and intermediate metastatic cell lines 530, 1F6, MV1 and M14. Me157 shows degradation of its mRNA, while no expression could be detected in the highly metastatic cell lines MV3 and BLM. Very weak expression was seen in 1F6m cells. The weak 1.0 kb band might be a homologues mRNA sequence or an alternative transcript. It could also be due to some aspecific hybridization. Sequencing of the 250 bp partial cDNA, containing the 3' poly-A tail, and subsequent homology searching only revealed significant homology with several human ESTs.

### Example 2

### Cloning of the full length cDNA

Using the partial 250 bp cDNA clone as a probe, a λZAP cDNA library of human melanoma cell line 530 was screened, in which marked expression of the 4.2 kb transcript was detected on Northern blot. A 3845 bp full length cDNA was isolated and sequenced (Fig. 2). The original 250 bp clone matched completely with the 3' part of the full length cDNA. The cDNA contained an open reading frame (ORF) of 687 amino acids including seven C-terminal transmembrane domains and a N-terminal signal peptide. The protein has a very high leucine and serine content of respectively 14.8 and 11.1%. It also has seven N-linked glycosylation sites in the extracellular part. Homology and domain searching using all kinds of databases and programs, from several Internet-servers, revealed a perfect homology with a lot of human ESTs from several types of both normal and tumor tissues. Highest homology with known genes was towards members of the secretin/calcitonin family of G-protein-coupled receptors, especially the EGF-TM7 subfamily. No functional clues from homology to known domains were found in the long N-terminal part. Because of its homology with the EGF-TM7 molecules the molecule was named TM7XN1.

### Homology search

Because of the strong homology of the C-terminal transmembrane domains the gene could be placed in the secretin/calcitonin family of G-protein-coupled receptors. TM7XN1 showed strongest homology with the EGF-TM7 family members CD97 and EMR1. Next to the 30% identity in the C-terminal part it also has a long extracellular N-terminal part, though it lacks the EGF binding domains which characterize the EGF-TM7 family. To check for other possibly interesting domains the MOTIFFINDER (http://www.motif.genome.ad.jp) was used. Besides several phosphorylation sites, seven N-glycosylation sites were found in the extracellular part. In the intracellular part, next to some phosphorylation sites a putative AMP-binding domain (a.a. 675-686) was predicted (Fig. 2). The PSORT II server (http://psort.nibb.ac.jp) predicted TM7XN1 to be present in the membrane of the endoplasmatic reticulum or in the plasma membrane.

To get more insight into the relationship between TM7XN1 and the secretin receptor and EGF-TM7 molecules TM7XN1 was compared with several other possible family members that were found previously and with some other proteins that were additionally found during homology searches with parts of TM7XN1. Some proteins of non-human origin were used in case no human homologue was known. The molecules from human origin are: calcitonin receptor (Gorn, A.H., et al., J. Clin. Invest. 90 (1992) 1726-1735), secretin receptor (Jiang, H., et al., Oncogene 11 (1995) 2477-2486), HE6 (human epididymis gene product 6) (Osterhoff, C., et al., DNA Cell Biol. 16 (1997) 379-389), BAI-1 (brain-specific angiogenesis inhibitor 1) (Nishimori, H., et al., Oncogene 15 (1997) 2145-2150), BAI-2 and BAI-3 (Shiratsuchi, T., et al., Cytogenet. Cell Genet. 79 (1997) 103-108), CD97 (Hamann, J., et al., J. Immunol. 155 (1995) 1942-1950), EMR1 (egf-like module containing mucin-like hormone receptor-like sequence 1) (Baud, V., et al., Genomics 26 (1995) 334-344), KIAA0279 (Nagase, T., et al., DNA Res. 3 (1996) 341-354), and R29368.2 (Mohrenweiser, H., et al., Cytogenet. Cell Genet. 74 (1996) 161-186). B0457.1 and F31D5.5 (Wilson, R., et al., Nature 368 (1994) 32-38), BOSS (bride of sevenless) (Hart, A.C., et al., Proc. Natl. Acad. Sci. USA 90(1993) 5047-5051), and B0286.2 (Wilson, R., et al., Nature 368 (1994) 32-38) are genes from Drosophila melanogaster, rat latrophilin (Lelianova, V.G., et al., J. Biol. Chem. 272 (1997) 21504-21508), and GRL101 from mollusc (Tensen, C.P., et al., Proc. Natl. Acad. Sci. USA 91 (1994) 4816-4820).

All sequences aligned have seven C-terminal transmembrane domains. The proteins vary markedly in length, with the secretin and calcitonin receptor (G-protein-coupled receptor family 2) being smaller than the other proteins shown. Peptide hormone receptors, like the secretin and calcitonin receptor, also have a relatively short N-terminus (<200 a.a.), containing their hormone binding domain. The recently described EGF-TM7 family (CD97 and EMR1), which is very homologous to the secretin receptor family, has a much longer extracellular domain containing several EGF binding domains (McKnight, A.J., and Gordon, S., Immunol. Today 17 (1996) 283-287). CD97 also has a RGD motif (Arg-Gly-Asp), which is thought to be involved in integrin-mediated cell-cell recognition (Hamann, J., et al., J. Immunol. 155 (1995) 1942-1950). The TM7XN1 protein is very homologous to the secretin receptor and to CD97 and EMR1, but lacks any known domain in its long N-terminus. Cleavable signal peptides of about 25 amino acids were found in all proteins of which the start of translation is known (von Heijne, G., Eur. J. Biochem. 133 (1983) 17-21), except for B0286.2. All aligned EGF- and LN-TM7s are substantially bigger than the peptide hormone receptors. Their N-terminus is longer than 300 bp and most proteins contain a so-called cystein-box: C-x₁₆₋₁₈-C-x₁₁-C-x-C (Osterhoff, C., et al., DNA Cell Biol. 16 (1997) 379-389). New EGF-TM7 molecules are KIAA0279 and R29368.2. KIAA0279 contains six EGF-binding domains, a leucin-zipper motif, and four cadherin binding domains. B0286.2 was previously described as a homologue without EGF binding domains, but using the MOTIFFINDER program (http://www.motif.genome.ad.jp/), one EGF domain could be identified. B0286.2 also has one additional transmembrane domain halfway its N-terminus. R29368.2 is a partial cDNA from which so far only part of the ORF, containing a cys-box and TM7 domain, is known. Because of its 50% identity with EMR1 and because the gene is also located on 19pl3, as are CD97 and EMR1, this gene is suspected to be an EGF-TM7 family member. Of the 'novel' LN-TM7 family, lacking EGF binding domains, some proteins have other known domains. BAI-1 and F31D5.5 have a RGD-motif, while BAI-2 contains a leucin-zipper motif and two additional TM domains. All BAI genes contain five thrombospondin type-1 repeats which are believed to be the domains involved in angiogenesis inhibition (Shiratsuchi, T., et al., Cytogenet. Cell Genet. 79 (1997) 103-108; Nishimori, H., et al., Oncogene 15 (1997) 2145-2150). GRL101 has twelve class A, cystein-rich, motifs present in the low density lipoprotein (LDL-RA) and six leucin rich repeats (LRR). Almost all LN- and EGF-TM7 molecules have a highly glycosylated mucin-like region within their N-terminal extracellular part between the membrane spanning-domain and the protein binding domain. This region is often lacking cysteine residues.

An alignment of the seven transmembrane domains and the cystein box preceding the first membrane domain shows that all proteins have the characteristic conserved cysteines in loop 2 and loop 4 which are thought to be necessary for stabilization of the transmembranal conformation of the G-protein-coupled receptors (Watson, S., and Arkinstall, S. (1994). "The G-protein Linked Receptor Facts Book," Academic Press, London). From the alignment of the cystein box a LN-TM7 specific protein consensus sequence for the cys-box can be distilled: C-x₂-W-x₄₋₁₆-W-x₄-C-x₁₁-C-x-C. Next to the consensus described earlier (Osterhoff, C., et al., DNA Cell Biol. 16 (1997) 379-389) also some very highly conserved tryptophane residues are present. Some less well-conserved but very frequent amino acids, like the FAVLM sequence, in the last part of the cys-box are found. The calcitonin receptor, secretin receptor, F31D5.5, BOSS, and GRL101 do not have a pronounced cystein box. Overall consensus sequence for the transmembrane domains are: TM1: G-x₈-L; TM₂: L; TM₃: C-x₁₆-W-x₃-E; TM4: G-x₃-P; TM5: -; TM6: W; TM7: F-x₆-QG. BOSS and GRL101 have a much lower homology compared to the other LN- and EGF-TM7 genes. LN-TM7 specific consensus sequences of the transmembrane domains are: TM1: G-x₇-CL; TM2: LC-x₉-FL-x-G; TM3: C-x₃-A-x₂-LH-x₃-L-x₂-F-x-W-x₃-E; TM4: G-x₃-P; TM5: -; TM6: TW; TM7: LF-x₃-N-x₂-QG-x₂-IF. No real specific EGF-TM7 conserved amino acids could be detected which lack in the LN-TM7 family. BAI-2 contains an extra transmembrane domain between TM5 and TM6.

### TM7XN1 expression profile

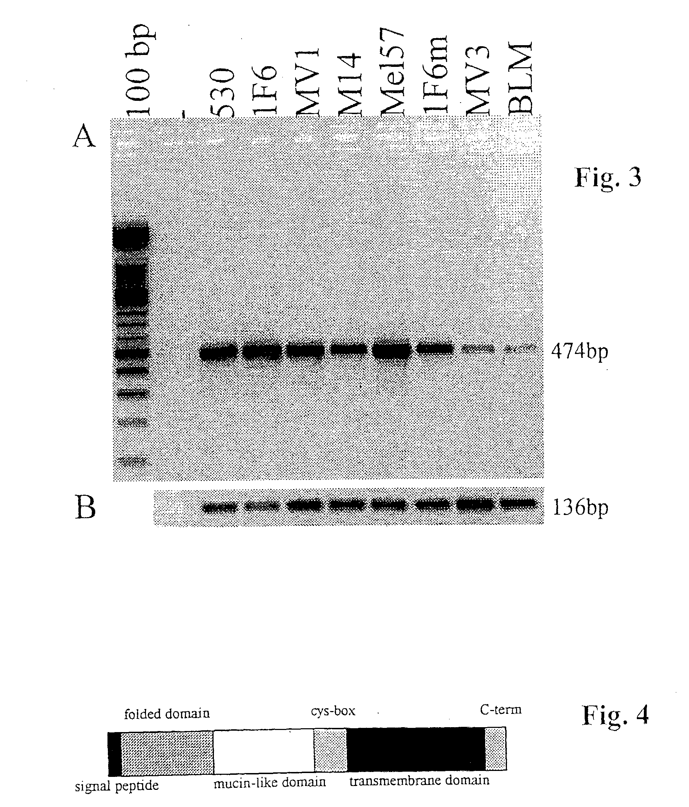

Expression of TM7XN1 was tested on a great number of cell types and tumor cell lines using RT-PCR. On a melanoma cell line panel with known metastatic behavior, there was found a marked downregulated expression in the highly metastatic cell lines MV3 and BLM compared to most of the non and intermediately aggressive cell lines (Fig. 3). Compared to the strong expression in 530, 1F6, MV1 and Me157, expression in M14 and lF6m seems somewhat lower. Though Northern blot was negative for MV3 and BLM (Fig. 1B) using RT-PCR some weak expression could be detected. RT-PCR on RNA isolated from corresponding xenografts of the panel matched completely with the results of the cultured cells. RT-PCR results on expression of TM7XN1 in all other cultured cells and cell lines tested are shown in Table 1.

**Table 1**

| Expression of TM7XN1 in human tumor cell lines, primary cell cultures, and tissues determined by RT-PCR | | | | | |
|---|---|---|---|---|---|
| RT-PCR expression category* | | | | | |
| tissue/cell type | ++ | + | +/- | - | total |
| melanoma cell lines | IF6; 451Lu; 518A2; 530; 603; A375m; A375p; Bowes; M24met; Me157; MKR; MV1; OCM1; OMM1; WM164 | 1F6m; E10; M14; ZKR | BLM; BRO; MV3; SKMe128 | | 23 |
| other tumor cell lines | A431; CaCo2; TH29; Molt4; Umssc2 | Hep/G2; HT1080 | U2OS | Daudi; Jurkat; K562; U937 | 12 |
| primary cell cultures | keratinocytes; naevus cells; endothelial cells; melanocytes | fibroblasts | PBMCs; pericytes; smooth muscle cells | dendritic cells | 9 |
| organ tissue | | kidney; prostrate; testis | bladder; brain; lung; thyroid gland; uterus | bone marrow; colon; heart; ileum; liver; lymph gland; pancreas; spleen; stomach | 17 |

| | | | | | |
|---|---|---|---|---|---|
| * The amounts of product present in Fig. 3A are representative for expression categories used. | | | | | |

All other human melanoma cell lines showed a varying expression. Only four of all tested melanoma cell lines (MV3, SKmel28, BLM and its parental cell line BRO) showed a weak expression. Furthermore, TM7XN1 was expressed in most cell lines derived from other tumor types. Only Daudi, Jurkat, K562, and U937 were negative. RT-PCR on RNA isolated from various human tissues showed highest expression in kidney, prostate and testis. Weak expression was seen in bladder, brain, lung, uterus and thyroid gland. No expression was found in bone marrow, colon, heart, ileum, liver, lymph node, pancreas, spleen, and stomach.

Expression was also studied in various lesions derived from all stages of the human melanocytic tumor progression. All lesions tested were positive. To get an impression whether expression in these lesions was due to specific expression in the melanocytic cells or to expression in other cell types present in the skin several primary cell cultures were tested. Strong expression was observed in keratinocytes, melanocytes, nevus cells and endothelial cells (Table 1). Some expression was present in fibroblasts, smooth muscle cells, pericytes, and isolated peripheral blood mononuclear cells (PBMCs). Dendritic cells (non-stimulated) were negative.

### Chromosomal localization

To check for homology with known sites in the human genome the Human Gene Map (NCBI) was looked into and it was found that TM7XN1 has perfect homology to an unidentified transcript. This STS (Sequence Tagged Site) was called STSG1704 and located on 16q13 between microsatelite markers D16S419 and D16S408 (65-72 cM). To confirm localization of TM7XN1 on chromosome 16, an intronspanning PCR on DNA of a human chromosome specific hybrid cell panel (hamster/human or mouse/human) was performed. There could only be detected the 528 bp specific product in the DNA sample of the chromosome 16 specific hybrid cell line.

### Example 3

### Antibodies against TM7XN1

Polypeptides aa 638-652 is synthesized and coupled to BSA. Polypeptide aa 2-315 is produced by recombinant means in E.coli as GST-fusion protein and purified by glutathione sepharose beads. Rabbits are interdermally immunized separately with these immunogens in a first immunization (500 µg immunogen, Freund's adjuvant) and with further intravenous boosts (500 pg immunogen, Freund's adjuvant). Test bleeds were done one week after each boost and binding was testes against the antigen of the immunogens and the full length TM7XN1 protein (with and without signal sequence).

### List of References

Albelda, S.M., Lab. Invest. 68 (1993) 4-17
Altschul, S.F., et al., J. Mol. Biol. (1990) 403-410
Aust, G. et al., Cancer Res. 57 (1997) 1798-1806
Ausubel I., Frederick M., Current Protocols in Mol. Biol. (1992), John Wiley and Sons, New York
Baud, V., et al., Genomics 26 (1995) 334-344
Bauer, D., et al., Nucleic Acids Res. 21 (1993) 4272-4280
Biggs, P.J., et al., Oncogene 12 (1996) 1375-1377
Buttner et al., Mol. Cell. Biol. 11 (1991) 3573-3583
Callard, D., et al., Biotechniques 16 (1994) 1096-1097
Callard, D., et al., Biotechniques 16 (1994) 1100-1103
Danen, E.H., et al., Melanoma Res. 6 (1996) 31-35 de Vries, T.J., et al., Cancer Res. 57 (1997) 3223-3229
Duncan, L.M., et al., Cancer Res. 58 (1998) 1515-1520
Ebnet, K., et al., Annu. Rev. Immunol. 14 (1996) 155-177
EP-A 0 063 879
EP-A 0 128 018
EP-A 0 173 251
EP-A 0 200 362
Geurts van Kessel, A.H., et al., Proc. Natl. Acad. Sci. USA 80 (1983) 3748-3752 Gorn, A.H., et al., J. Clin. Invest. 90 (1992) 1726-1735
Hamann, J., et al., Genomics 32 (1996) 144-147
Hamann, J., et al., J. Immunol. 155 (1995) 1942-1950
Hames, B.D., Higgins, S.G., Nucleic acid hybridisation - a practical approach (1985) IRL Press, Oxford, England
Harlow and Lane eds., Antibodies: A laboratory manual (1988), Cold Spring Harbor Laboratories Press
Jiang, H., et al., Oncogene 11 (1995) 2477-2486
Juhasz, I., et al., Am. J. Pathol. 143 (1993) 528-537
Kohler and Milstein, Nature 256 (1975) 495-497
Lee, J.H., et al., Int. J. Cancer 71 (1997) 1035-1044
Lelianova, V.G., et al., J. Biol. Chem. 272 (1997) 21504-21508
Liang, P., and Pardee, A.B., Science 257 (1992) 967-971
Lockshin, A., et al., Cancer Res. 45 (1985) 345-450
Luyten, G.P., et al., Int. J. Cancer 66 (1996) 380-387
Madaule, P., et al., FEBS Lett. 377 (1995) 243-248
McKnight, A.J., and Gordon, S., Immunol. Today 17 (1996) 283-287
McKnight, A.J., and Gordon, S., J. Leukoc. Biol. 63 (1998) 271-280
Mohrenweiser, H., et al., Cytogenet. Cell Genet. 74 (1996) 161-186
Nagase, T., et al., DNA Res. 3 (1996) 341-354
Nishimori, H., et al., Oncogene 15 (1997) 2145-2150
Osterhoff, C., et al., DNA Cell Biol. 16 (1997) 379-389
Pardee, A.B., Advances in Cancer Res. 65 (1994) 213-227
Ponta, H., et al., Biochem. Biophys. Acta 1198 (1994) 1-10
Probst, W.C., et al., DNA Cell Biology 11 (1992) 1-20
Quax, P.H., et al., Cancer Res. 50 (1990) 1488-1494
Sambrook et al., Molecular Cloning: A laboratory manual (1989) Cold Spring Harbor Laboratory Press, New York, USA
Scheibenbogen, C., et al., Int. J. Cancer 54 (1993) 494-498
Shen, R., et al., Proc. Natl. Acad. Sci. USA 92 (1995) 6778-6782
Shiratsuchi, T., et al., Cytogenet. Cell Genet. 79 (1997) 103-108
Tensen, C.P., et al., Proc. Natl. Acad. Sci. USA 91 (1994) 4816-4820
USP 2915082 van Groningen, J.J., et al., Cancer Res. 55 (1995) 6237-6243 van Lier, R.A., et al., Immunol. Lett. 54 (1996) 185-187
Varner, J.A., and Cheresh, D.A., Curr. Opin. Cell Biol. 8 (1996) 724-730
Velculescu, V.E., et al., Science 270 (1995) 484-487 von Heijne, G., Eur. J. Biochem. 133 (1983) 17-21
Wahl, G.M., et al., Proc. Natl. Acad. Sci. USA 76 (1979) 3683-3687
Watson, S., and Arkinstall, S. (1994). "The G-protein Linked Receptor Facts Book," Academic Press, London
Westphal, J.R., et al., Br. J. Cancer 76 (1997) 561-570
Weterman, M.A., et al., Lab. Invest. 70 (1994) 593-608
Wilson, R., et al., Nature 368 (1994) 32-38
WO 89/06698
Yeatman, T.J., et al., Nucleic Acids 23 (1995) 4007-4008
Zhang, L., et al., Sciene 276 (1997) 1268-1272

## Claims

1. An isolated nucleic acid molecule (TM7XN1) which is down-regulated during tumor progression and/or metastasis, wherein the nucleic acid is selected from the group consisting of
a) a nucleic acid having the sequence SEQ ID NO:1,
b a nucleic acid which hybridizes under stringent conditions with a nucleic acid fragment coding for amino acids 2-315 of said nucleic acid of a) or the complementary fragment or
c) a nucleic acid which due to the degeneracy of the genetic code encodes a polypeptide encoded by one of the foregoing nucleic acids.

2. An isolated nucleic acid molecule which codes for a polypeptide of SEQ ID NO:2 or a fragment thereof containing amino acids 2-315 of SEQ ID NO:2, said polypeptide inducing tumor progression and/or metastasis.

3. A recombinant expression vector which is suitable for the expression of a nucleic acid molecule as claimed in claim 1 or 2.

4. A recombinant polypeptide which is down-regulated during tumor progression and/or metastasis and
a) is coded by the DNA sequence shown in SEQ ID NO:1 or
b) is coded by a DNA sequence which hybridizes under stringent conditions with the DNA fragment coding for amino acids 2-315 of SEQ ID NO:1 or the complementary sequence.

5. A method for the production of a polypeptide which is down-regulated during tumor progression and/or metastasis, by expressing an exogenous DNA in prokaryotic or eukaryotic host cells and isolation of the desired polypeptide, wherein the polypeptide
a) is coded by the DNA sequence shown in SEQ ID NO: 1 or
b) is coded by a DNA sequence which hybridizes under stringent conditions with the DNA sequence shown in SEQ ID NO:1 or the complementary sequence.

6. A method for the detection of the metastatic potential of a tumor cell comprising
a) incubating a sample of body fluid of a patient suffering from cancer, of melanoma cancer cells, or of a cell extract or cell culture supernatant of said melanoma cancer cells, whereby said sample contains nucleic acids with a nucleic acid probe which is selected from the group consisting of
(i) the nucleic acid shown in SEQ ID NO:1 or a nucleic acid which is complementary thereto and
(ii) nucleic acids which hybridize with one of the nucleic acids from (i) and
b) detecting the hybridization by means of a further binding partner of the nucleic acid of the sample and/or the nucleic acid probe.

7. The method of claim 6, wherein the nucleic acid to be detected is amplified before the detection.

8. A method for the production of antibodies against a polypeptide which is down-regulated during tumor progression and/or metastasis (TM7XN1), wherein the polypeptide of claim 4 or a fragment thereof is coupled to an immunogen, animals are immunized with said fused immunogen and the antibodies specific for binding to TM7XN1 are isolated from body fluid of said animals.
